# EUROPÄISCHE PATENTSCHRIFT

(11) **EP 0 899 642 B1**
(45) Veröffentlichungstag und Bekanntmachung des Hinweises auf die Patenterteilung: **14.08.2002**
(21) Anmeldenummer: 98109929.4
(22) Anmeldetag: 30.05.1998
(51) Int. Cl.: G05D 21/02, E04H 4/12

(54) **Mess-, Regel- und Dosierstation**
Apparatus for measuring, controlling and dosing
Dispositif de mesure, de régulation et de dosage

(30) Priorität: 27.08.1997 DE 29715378 U
(43) Veröffentlichungstag der Anmeldung: 03.03.1999
(73) Patentinhaber: Dinotec GmbH Wassertechnologie und Schwimmbadtechnik, 63477 Maintal (DE)
(72) Erfinder: Schminke, Dieter, 60598 Frankfurt am Main (DE)
(74) Vertreter: KEIL & SCHAAFHAUSEN

(56) Entgegenhaltungen:
- WO-A-96/06284
- FR-A- 2 491 530
- FR-A- 2 585 148
- FR-A- 2 715 865
- US-A- 4 033 871
- US-A- 4 945 939
- F. OEHME: "Automatisches Messen und Regeln der Konzentration von Bleichbädern" TEXTIL VEREDLUNG, Bd. 1, Nr. 7, August 1966, Seiten 350-359, XP002086375
- C.H. RUPP: "Ab 700 Millivolt haben Keime keine Chance" SCHWIMMBAD UND SAUNA., Bd. 16, Nr. 6/7, Juni 1984 - Juli 1984, Seiten 70-77, XP002086376 STUTTGART DE

## Beschreibung

Die Erfindung betrifft eine Meß-, Regel- und Dosierstation zur Aufbereitung (Entkeimung, Algenverhinderung, pH-Wert-Einstellung und dergleichen) von wäßrigen Lösungen gemäß dem Oberbegriff des Anspruchs 1.

Zur Entkeimung und Algenverhütung von Schwimmbadwasser wird seit langem Chlor, seit mehreren Jahren aber auch verstärkt Wasserstoffperoxid (Aktivsauerstoff) eingesetzt. Um die Hautverträglichkeit des Wassers zu erhöhen, wird ferner darauf geachtet, daß das Wasser einen hautneutralen pH-Wert von etwa 7,2 aufweist und dieser Wert durch die entsprechende Zugabe von Regulatoren möglichst konstant gehalten. Alle diese Produkte werden dem Schwimmbadwasser entweder manuell oder über Dosierpumpen zugesetzt. Die Dosierpumpen werden über eine Zeitschaltung oder, um eine bessere Genauigkeit und schnellere Reaktionszeiten zu erreichen, über eine Regeleinrichtung gesteuert, die die entsprechenden Wasserwerte ermittelt und auf dieser Basis die Zugabe von Entkeimungsmittel etc. festlegt. Die von der Meßzelle ermittelten Werte werden hierbei in einer Regeleinrichtung analysiert und in Steuerimpulse umgewandelt, die dann an die extern vorgesehenen Dosierpumpen für die Zugabe von Entkeimungsmittel etc. weitergegeben werden.

Die gattungsgemäße US 4,945,939 beschreibt ein pH-Wert-Steuerungssystem zur automatischen Steuerung des pH-Wertes in einem Flüssigkeitsreservoir durch Zugabe von den pH-Wert beeinflussenden Flüssigkeiten wie Säuren oder Basen. Dazu wird ein Teil der zu überwachenden Flüssigkeit durch einen pH-Wert-Detektor und einen Injektor geleitet, in dem eine von einem Steuerungscomputer bestimmte Menge der den pH-Wert beeinflussenden Flüssigkeit zugegeben wird. Zur Dosierung der zuzuführenden Flüssigkeit wird diese aus einem Steuerungstank durch Öffnen eines Ventils in eine Steuerungskammer definierter Größe eingefüllt. Sobald sich die vorbestimmte Menge der den pH-Wert beeinflussenden Flüssigkeit in der Steuerungskammer befindet, wird das Zufuhrventil in die Steuerungskammer geschlossen und das Auslaßventil geöffnet. Dieser Vorgang wird durch eine Computersteuerung überwacht.

Aus der US 4,033,871 ist ein vergleichbares Steuerungssystem für eine kontinuierliche Überwachung und Steuerung des pH-Wertes und der freien Halogenkonzentration in Schwimmbadwasser beschrieben, bei dem die Messzellen außerhalb des Gehäuses angeordnet sind und Solenoidventile zum Öffnen und Schließen der Reservoirs mit normaler Wechselspannung betrieben werden. Zum Schalten der Pumpen sind Hochleistungstransistoren und Relais vorgesehen, welche einen Stromschalter schließen oder öffnen und dadurch die Solenoidventile steuern.

Aus der FR 2 715 865 A1 ist bekannt, eine Pumpe für ein Filtersystem, welche in einem Wasserreservoir angeordnet wird, mit Niedrigspannung zu betreiben. Diese Pumpe sorgt für den kontinuierlichen Durchfluß der zu filternden Flüssigkeit durch das Filter.

Schließlich beschreibt die FR 2 491 530 A die Verwendung von Schlauchpumpen bei Anlagen zur automatischen Entkeimung von Schwimmbadwasser.
Der Aufbau der bekannten Meß-, Regel- und Dosiersituation erfordert jedoch eine Vielzahl von Einzelteilen, die in aufwendiger Weise am Einsatzort, d.h. im Schwimmbad, montiert werden müssen. Hierbei muß die Regeleinrichtung zum einen aufwendige Ansteuerungsbauteile aufweisen, um die unterschiedlichen Pumpentypen, die mit der Regeleinrichtung verwendet werden, korrekt ansteuern zu können. Außerdem erfordern die Dosierpumpen, die mit 220 V-Wechselspannung betrieben werden, einen gesonderten Stromanschluß, was aufgrund der Feuchtigkeit in Schwimmbädern immer eine Gefährdungsquelle darstellt.

Aufgabe der Erfindung ist es daher, den Aufbau einer derartigen Meß-, Regelund Dosierstation zu vereinfachen.

Diese Aufgabe wird bei der Erfindung mit den Merkmalen des Anspruchs 1 gelöst.

Durch die serienmäßige Integration der Dosierpumpen in das Gehäuse der Regeleinrichtung wird der Installationsaufwand für ein Schwimmbad erheblich reduziert. Alle wesentlichen Anschlüsse können bereits werkseitig vormontiert werden. Da die Dosiereinrichtungen mit derselben Niederspannung arbeiten, wie die Regeleinrichtung, kann auf eine gesonderte Stromquelle für die Dosiereinrichtung verzichtet werden. Vor allem aber kann auf Relais oder ähnliche Zwischenstufen zur Übertragung der Dosiersignale von der Regeleinrichtung auf die Pumpen verzichtet werden, da die Pumpen direkt mit ihrer Versorgungsspannung aus der Elektronik angesteuert werden können. Erfindungsgemäß weist die Meßzelle einen Platinstift zur Bestimmung der Redoxpotentialspannung als Referenzgröße für den Entkeimungsmittelgehalt auf. Dabei ist vorgesehen, daß die Redoxpotentialspannung in einem Anzeigefenster angezeigt wird, das in Abhängigkeit von den Bedingungen im Schwimmbad, einer eventuellen Verschmutzung der Elektroden bzw. des Platinstiftes oder dem Wohlbefinden des Benutzers verschiebbar ist, um den Sollwert des Entkeimungsmittelgehaltes zu korrigieren. Hierbei wird berücksichtigt, daß der Platinstift im Gegensatz zu den herkömmlichen Elektroden zum Säubern nicht herausgenommen werden kann. Er ließe sich allenfalls durch ein Ausspülen der Zelle mit Säure "säubern". Dies wird durch die Verschiebung des Anzeigefensters vermieden. Gemäß einer bevorzugten Ausgestaltung der Erfindung ist in dem Gehäuse sowohl eine Dosiereinrichtung zur Förderung eines Desinfektionsmittels als auch eine Dosiervorrichtung zur Zugabe eines pH-Wert-Regulators vorgesehen, so daß gleichzeitig eine Dosierung beider wesentlicher Wasserpflegemittel erfolgen kann.

Eine besonders einfache und kostengünstige Gestaltung der erfindungsgemäßen Meß-, Regel- und Dosierstation ergibt sich, wenn die Dosiereinrichtung eine Schlauchdosierpumpe mit einer Niederspannungs-Antriebseinheit aufweist.

Um dem Benutzer die Arbeit weitgehend abzunehmen und Fehlbedienungen zu verhindern, wird die Zugabe des Desinfektionsmittels etc. weitgehend automatisch gesteuert. Hierzu sind erfindungsgemäß in der Regeleinrichtung Sollwerte für die Wasserqualität gespeichert, wobei die Regeleinrichtung mit Hilfe eines Mikroprozessors bei Abweichungen der gemessenen Istwerte von den Sollwerten selbsttätig die Zugabe von Desinfektionsmittel, pH-Wert-Regulatoren und dergleichen so steuert, daß der Istwert des Wassers dem Sollwert angeglichen wird.

Zusätzlich weist das Gehäuse jedoch vorzugsweise eine Anzeigeeinheit auf, um visuell anzuzeigen, ob die gemessenen Istwerte zu hoch, zu niedrig oder auf dem Niveau der Sollwerte liegen, und dadurch dem Benutzer eine Kontrolle zu ermöglichen.

Hierbei kann die Zugabe der Wasserpflegemittel nach dem persönlichen Wohlbefinden gestaltet werden, da erfindungsgemäß an dem Gehäuse Einstellschalter vorgesehen sind, über die die Sollwerte veränderbar sind. Durch Anpassung der Sollwerte steuert die Regeleinrichtung die Zugabe der Wasserpflegemittel automatisch so, daß sie dem gewünschten Sollwert entsprechen.

Die Einstellschalter können außerdem zur Festlegung der Grundeinstellung (Kalibrieren) des Gerätes verwendet werden.

Weiterbildungen, Vorteile und Anwendungsmöglichkeiten der Erfindung ergeben sich auch aus der nachfolgenden Beschreibung von Ausführungsbeispielen und der Zeichnung.

Es zeigen:
- Fig. 1: Schematisch eine Meß-, Regel- und Dosierstation gemäß der vorliegenden Erfindung mit einer ersten Meßzelle,
- Fig. 2: schematisch eine andere Meßzelle zur Verwendung mit der Station gemäß Fig. 1, und
- Fig. 3: schematisch eine dritte Meßzelle zur Verwendung mit der Station gemäß Fig. 1.

Die in der Zeichnung dargestellte Meß-, Regel- und Dosierstation 1 besteht im wesentlichen aus einer Meßzelle 2 und einer Regeleinrichtung 3, die ggf. werksseitig auf einer Montageplatte 4 vormontiert sind. Somit müssen am Einsatzort im Schwimmbad nur noch die Schläuche für die Zu- und Abfuhr von Wasser, Entkeimungsmittel, pH-Wert-Regulator etc. angeschlossen werden.

In der Meßzelle 2 wird das über einen Zulauf 5 zugeführte und über einen Ablauf 6 abgeführte Schwimmbadwasser kontinuierlich analysiert. Je nach anzuwendendem Entkeimungsverfahren werden unterschiedliche Meßzellen verwendet. So gibt es Meßzellen für die Messung von freiem Chlor, Redox-Spannung und pH-Wert; nur Chlor und pH-Wert; Redox-Spannung und pH-Wert; und Wasserstoffperoxid und pH-Wert.

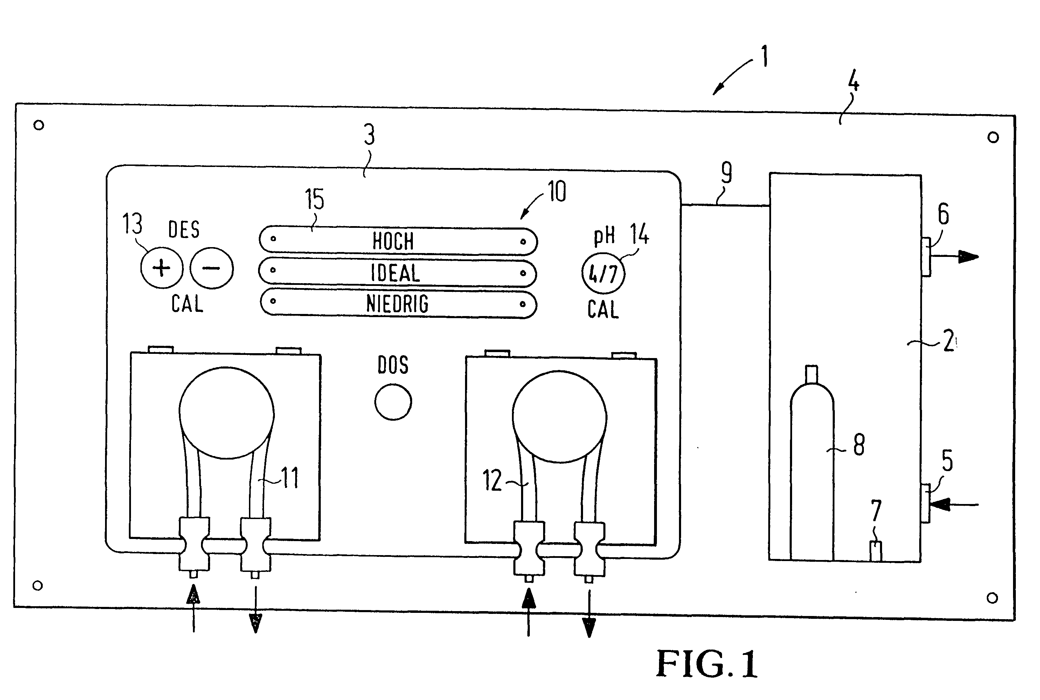

Mit Bezug auf Fig. 1 wird nachfolgend eine Meßzelle 2 zur Erfassung der Redox-Spannung und des pH-Wertes erfolgt. Hierzu ist in der Meßzelle 2 ein Platinstift 7 vorgesehen, über den an Stelle der bisher üblichen Redox-Elektrode die Redoxmessung zur Bestimmung des Desinfektionsmittelgehaltes durchgeführt wird. Über eine Glas-pH-Elektrode 8 wird gleichzeitig der pH-Wert des Schwimmbadwassers ermittelt.

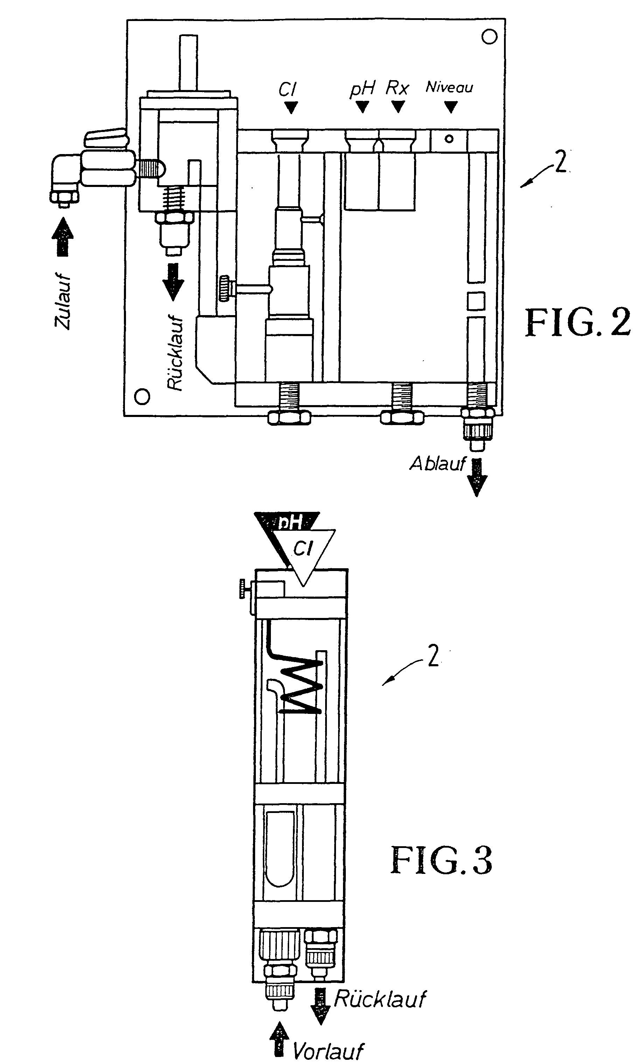

In Fig. 2 ist schematisch eine Meßzelle 2 zur Aufnahme von 4 Elektroden und integrierter Gegenelektrode für die potentiostatische Chlormessung dargestellt. Die Meßzelle 2 enthält Elektroden für Chlor, pH-Wert, Redox und zur Meßwasserüberwachung. Durch das potentiostatische Meßprinzip wird eine selektive Messung von freiem Chlor ermöglicht.

Fig. 3 zeigt schematisch eine Meßzelle 2 mit einer Elektrode und integrierter Gegenelektrode sowie einer pH-Elektrode und integrierter Durchlaufüberwachung. Diese im geschlossenen Kreislauf arbeitende Meßzelle ist besonders für den Inlinebetrieb mit Meßwasserrückführung geeignet.

Die ermittelten Meßwerte werden über eine Signalleitung 9 zu der in einem Gehäuse 10 vorgesehenen Regeleinrichtung 3 weitergeleitet.

In dieser mit Niedrigspannung von 24 V betriebenen Regeleinrichtung 3 werden die Meßwerte weiterverarbeitet und mit voreingestellten Sollwerten für die Wasserpflegemittel verglichen. Stellt die Regeleinrichtung 3 fest, daß die gemessenen Istwerte von den voreingestellten Sollwerten abweichen, so werden ebenfalls in dem Gehäuse 10 angeordnete Schlauchdosierpumpen 11, 12 für Desinfektionsmittel bzw. pH-Wert-Regulator so angesteuert, daß sich die Istwerte des entsprechenden Wasserpflegemittels wieder den Sollwerten angleichen. Die Dosierpumpen 11, 12 werden mit der gleichen Niederspannung angetrieben wie die Regeleinrichtung 3, so daß sie direkt über die Versorgungspannung der Elektronik angesteuert werden können. Eine gesonderte Stromzufuhr zu den Dosierpumpen 11, 12 ist nicht erforderlich.

Zum erstmaligen Einstellen (Kalibrieren) der Station 1 sind Einstellschalter 13, 14 vorgesehen, über die die Zugabe des Desinfektionsmittels und des pH-Wert-Regulators getrennt einstellbar ist. Nach Kalibrierung des Meß-, Regel- und Dosierstation 1 wird dem Benutzer über eine Leuchtdioden-Anzeigeeinheit 15 der Zustand des Schwimmbadwassers angezeigt. Über Leuchtdioden für die Entkeimung bzw. den pH-Wert wird angezeigt, ob der Istwert zu hoch, ideal oder zu niedrig ist. Bei einer Abweichung vom Idealwert steuert die Regeleinrichtung 3 die Zugabe des Entkeimungsmittels bzw. pH-Wert-Regulators mit Hilfe eines Mikroprozessors automatisch so, daß der Istwert wieder dem Sollwert angenähert wird. Sofern der Benutzer die Wasserqualität seinem persönlichen Wohlbefinden individuell anpassen will, können die Sollwerte über die Einstellschalter 13, 14 verändert werden.

Bei der Redoxmessung wird die Redoxspannung nicht als absoluter Wert sondern nur als Referenzgröße für den Entkeimungsmittelgehalt angegeben. Es wird ein Anzeigefenster von bspw. 200 mV innerhalb des Gesamtmeßbereichs der Redoxpotentialspannung von 0-1000 mV angezeigt. Die Einstellung auf den gewünschten Idealwert kann durch Verschieben des Anzeigefensters, das bspw. über eine Leuchtdiodenkette "Entkeimung" angezeigt wird, erfolgen. Beginnt das Anzeigefenster bspw. bei 0 mV Redoxspannung, so endet die Anzeige bei 200 mV. Der Schaltpunkt für die Desinfektionsmitteldosierung liegt dann bei etwa 100 mV. Diese Referenzgrößenanalyse reicht aus, da in der Praxis die Redoxspannung bei gleichem Entkeimungsmittelgehalt von Bad zu Bad variieren kann. Damit ist auch die Nutzung des Platinstiftes 7 ausreichend und auf die bisher erforderliche Elektrode kann verzichtet werden. Durch die Verschiebung des Anzeigefensters kann ein eventuell entstehender Belag auf dem Platinstift 7, der die Intensität der Messung beeinträchtigen würde, kompensiert werden, ohne den Platinstift 7 in der Meßzelle 2 aufwendig reinigen zu müssen.

Die vorliegende Meß-, Regel- und Dosierstation 1 läßt sich somit auf besonders einfache Weise am Einsatzort anbringen, ohne daß die Gefahr von Fehlanschlüssen bestünde. Die Gestaltung der Station 1 wird dadurch wesentlich vereinfacht, daß die in dem gemeinsamen Gehäuse 10 angeordnete Regeleinrichtung 3 und Dosierpumpen 11, 12 über eine gemeinsame Stromversorgung angetrieben werden. Hierdurch wird insbesondere der Aufbau der Regeleinrichtung 3 wesentlich vereinfacht, da auf zusätzliche Ansteuerungsbauteile, wie Relais oder ähnliche Zwischenstufen, verzichtet und die Pumpen 11, 12 direkt mit ihrer Versorgungspannung aus der Elektronik angesteuert werden können. Die Bedienung der Station 1 ist über die Einstellschalter 13, 14 in Verbindung mit der Anzeigeeinheit 15 denkbar einfach.

### Bezugszeichenliste:

- 1: Meß-, Regel- und Dosierstation
- 2: Meßzelle
- 3: Regeleinrichtung
- 4: Montageplatte
- 5: Zulauf
- 6: Ablauf
- 7: Platinstift
- 8: pH-Wert-Elektrode
- 9: Signalleitung
- 10: Gehäuse
- 11, 12: Dosierpumpen
- 13, 14: Einstellschalter
- 15: Anzeigeeinheit

## Patentansprüche

1. Meß-, Regel- und Dosierstation zur Aufbereitung (Entkeimung, Algenverhinderung, pH-Wert-Einstellung etc.) von wäßrigen Lösungen, insbesondere zur Wasseraufbereitung in Schwimmbädern, wobei in einer als Durchlaufarmatur oder dergleichen ausgebildeten Meßzelle (2) Daten der aufzubereitenden wäßrigen Lösung (Schwimmbadwasser) ermittelt und in Meßsignale umgewandelt werden, und wobei in einem Gehäuse (10) sowohl eine Regeleinrichtung (3) zur Auswertung und Weiterverarbeitung der von der Meßzelle (2) gelieferten Meßsignale als auch wenigstens eine Dosiereinrichtung (11, 12) zur Förderung eines Desinfektionsmittels, pH-Wert-Regulators oder dergleichen aufgenommen sind, **dadurch gekennzeichnet, daß** die Regeleinrichtung (3) und die Dosiereinrichtung(en) (11, 12) mit Niederspannung, insbesondere einer Spannung aus 24 V, betrieben werden, daß die wenigstens eine Dosiereinrichtung (11, 12) über die Regeleinrichtung (3) ohne Zwischenschaltung von Relais oder ähnlichen Zwischenstufen direkt mit der Versorgungsspannung aus der Elektronik angesteuert wird, daß die Meßzelle (2) einen Platinstift (7) zur Bestimmung der Redoxpotentialspannung als Referenzgröße für den Entkeimungsmittelgehalt aufweist und daß die Redoxpotentialspannung in einem Anzeigefenster innerhalb des Gesamtbereichs der Redoxpotentialspannung angezeigt wird, das in Abhängigkeit von den Bedingungen im Schwimmbad, einer eventuellen Verschmutzung des Platinstiftes (7) oder dem Wohlbefinden des Benutzers verschiebbar ist, um den Sollwert des Entkeimungsmittelgehaltes zu korrigieren.

2. Station nach Anspruch 1, **dadurch gekennzeichnet, daß** in dem Gehäuse (8) eine Dosiereinrichtung (11) zur Förderung eines Desinfektionsmittels und eine Dosiervorrichtung (12) zur Zugabe eines pH-Wert-Regulators vorgesehen sind.

3. Station nach Anspruch 1 oder 2, **dadurch gekennzeichnet, daß** die Dosiereinrichtung (11, 12) eine Schlauchdosierpumpe mit einer Niederspannungs-Antriebseinheit aufweist.

4. Station nach einem der Ansprüche 1 bis 3, **dadurch gekennzeichnet, daß** in der Regeleinrichtung (3) Sollwerte für die Wasserqualität gespeichert sind, und daß die Regeleinrichtung (3) mit Hilfe eines Mikroprozessors bei Abweichungen der gemessenen Istwerte von den Sollwerten selbsttätig die Zugabe von Desinfektionsmittel, pH-Wert-Regulatoren oder dergleichen so steuert, daß der Istwert des Wassers dem Sollwert angeglichen wird.

5. Station nach Anspruch 4, **dadurch gekennzeichnet, daß** das Gehäuse (10) eine Anzeigeeinheit (15) aufweist, um visuell anzuzeigen, ob die gemessenen Istwerte zu hoch, zu niedrig oder auf dem Niveau der Sollwerte liegen.

6. Station nach einem der Ansprüche 1 bis 5, **dadurch gekennzeichnet, daß** an dem Gehäuse (10) Einstellschalter (13, 14) vorgesehen sind, über die die Sollwerte veränderbar sind.

## Claims

1. Measuring, control and metering station for the treatment (sterilisation, algae control, pH adjustment, etc.) of aqueous solutions, in particular for the treatment of water in swimming pools, in which data for the aqueous solution to be treated (swimming pool water) is determined in a measuring cell (2) in the form of a flow cell assembly or the like and is converted into measuring signals, and in which both a control device (3) for the evaluation and further processing of the measuring signals supplied by the measuring cell (2) and at least one metering device (11, 12) for supplying a disinfectant, pH regulator or the like are accommodated in a housing (10), **characterised in that** the control device (3) and the metering device(s) (11, 12) are operated with a low voltage, in particular a voltage of 24 V, that the at least one metering device (11, 12) is controlled directly with the supply voltage from the electronic system via the control device (3) without the interconnection of relays or similar intermediate stages, that the measuring cell (2) has a platinum pin (7) for determining the redox potential as a reference variable for the sterilising agent content and that the redox potential is displayed in a display window within the total range of the redox potential which can be displaced as a function of the conditions in the swimming pool, any contamination of the platinum pin (7) or the well-being of the user in order to correct the setpoint of the sterilising agent content.

2. Station according to claim 1, **characterised in that** a metering device (11) for supplying a disinfectant and a metering device (12) for adding a pH regulator are provided in the housing (10).

3. Station according to claim 1 or claim 2, **characterised in that** the metering device (11, 12) has a peristaltic metering pump with a low-voltage drive unit.

4. Station according to one of claims 1 to 3, **characterised in that** setpoints for the water quality are stored in the control device (3) and that the control device (3) automatically controls the addition of disinfectant, pH regulators or the like with the aid of a microprocessor if the actual measured values deviate from the setpoints, in such a manner that the actual value of the water is adapted to the setpoint.

5. Station according to claim 4, **characterised in that** the housing (10) has a display unit (15) in order to visually display whether the actual measured values are too high, too low or at the setpoints.

6. Station according to one of claims 1 to 5, **characterised in that** adjusting switches (13, 14) by means of which the setpoints can be varied are provided on the housing (10).

## Revendications

1. Poste de mesure, de réglage et de dosage pour le traitement (désinfection, prévention des algues, régulation de la valeur du pH, etc.) de solutions aqueuses, en particulier pour le traitement de l'eau dans des piscines, dans lequel des données de la solution aqueuse à traiter (eau de la piscine) sont déterminées et transformées en signaux de mesure dans une cellule de mesure (2) en forme d'armature à flux continu ou similaire, et dans lequel sont logés dans un boîtier (10) à la fois un dispositif de réglage (3) pour l'évaluation et le traitement des signaux de mesure fournis par la cellule de mesure (2) et au moins un dispositif de dosage (11, 12) pour l'acheminement d'une substance désinfectante, d'un régulateur de la valeur de pH ou d'un produit similaire, **caractérisé en ce que le** dispositif de réglage (3) et le ou les dispositifs de dosage (11, 12) sont alimentés par une basse tension, en particulier par une tension de 24 V, **en ce que** le au moins un dispositif de dosage (11, 12) est commandé par le biais du dispositif de réglage (3) directement avec la tension d'alimentation à partir de l'électronique sans interposition de relais ou de dispositifs intermédiaires similaires, **en ce que** la cellule de mesure (2) comprend une broche de platine (7) pour la détermination de la tension du potentiel redox comme valeur de référence pour la teneur en substance désinfectante et **en ce que** la tension du potentiel redox est affichée dans une fenêtre d'affichage à l'intérieur de la marge globale de la tension redox et qui peut être décalée en fonction des conditions dans la piscine, d'un éventuel encrassement de la broche de platine (7) ou du bien-être de l'utilisateur afin de corriger la valeur théorique de la teneur en substance désinfectante.

2. Poste selon la revendication 1, **caractérisé en ce que** sont prévus dans le boîtier (10) un dispositif de dosage (11) pour l'acheminement d'une substance désinfectante et un dispositif de dosage (12) pour l'ajout d'un régulateur de la valeur de pH.

3. Poste selon l'une des revendications 1 ou 2, **caractérisé en ce que** le dispositif de dosage (11, 12) comprend une pompe péristaltique de dosage avec une unité d'entraînement à basse tension.

4. Poste selon l'une des revendications 1 à 3, **caractérisé en ce que** des valeurs théoriques pour la qualité de l'eau sont mémorisées dans le dispositif de réglage (3) et **en ce que** le dispositif de réglage (3) commande lui-même, à l'aide d'un microprocesseur, l'ajout de substance désinfectante, de régulateurs de la valeur de pH ou de produits similaires en cas de déviations des valeurs réelles mesurées par rapport aux valeurs théoriques, de telle sorte que la valeur réelle de l'eau est mise en conformité avec la valeur théorique.

5. Poste selon la revendication 4, **caractérisé en ce que** le boîtier (10) comprend une unité d'affichage (15) pour afficher visuellement si les valeurs réelles mesurées sont trop élevées, trop basses ou au niveau des valeurs théoriques.

6. Poste selon l'une des revendications 1 à 5, **caractérisé en ce que** des commutateurs de réglage (13, 14) sont prévus sur le boîtier (10) et à l'aide desquels les valeurs théoriques peuvent être modifiées.
